# EUROPEAN PATENT APPLICATION

(11) **EP 4 656 062 A1**
(43) Date of publication of application: **03.12.2025**
(21) Application number: 24747337.4
(22) Date of filing: 24.01.2024
(51) Int. Cl.: A23L 27/00, A61K 31/353, A61K 47/02, A61K 47/18

(54) **SALTY TASTE/UMAMI ENHANCER**

(30) Priority: 25.01.2023 JP 2023009728
(71) Applicant: AJINOMOTO CO., INC., Chuo-ku Tokyo 104-8315 (JP)
(72) Inventor: KITAJIMA, Seiji, Kawasaki-shi, Kanagawa 210-8681 (JP); KANNO, Kyoko, Kawasaki-shi, Kanagawa 210-8681 (JP)
(74) Representative: Strehl & Partner mbB
(86) International application number: PCT/JP2024/002076
(87) International publication number: WO 2024/158013

(57) **Abstract**

In the present application, an agent for enhancing saltiness and/or umami of an oral ingestion product containing a component imparting saltiness and/or a component imparting umami, the agent containing one or two or more flavonoid components selected from naringenin, luteolin, pinocembrin, liquiritigenin, apigenin, and acacetin, or salts thereof, as an active ingredient, and the like are disclosed as embodiments thereof.

## Description

### [Technical Field]

The present invention relates to a saltiness and/or umami enhancer containing a flavonoid component such as naringenin and the like as an active ingredient, and the like.

### [Background Art]

Saltiness and umami are important elements of the five basic tastes, along with sweetness, sourness, and bitterness, and impartment or enhancement thereof is extremely important for the taste of foods, and the like.

Sodium chloride is used in foods and the like for various purposes such as imparting saltiness and the like. Since excessive intake of sodium chloride increases the risk of causing hypertension, cardiac disease or the like, and because of the recent health consciousness, efforts have been conventionally made to reduce the amount of sodium chloride intake. The amount of sodium chloride intake itself can be reduced by reducing the amount of sodium chloride used (i.e., sodium chloride reduction) when manufacturing foods or the like. In sodium chloride-reduced foods or the like, the shortage of saltiness sometimes reduces a sense of satisfaction. Therefore, various techniques for enhancing the saltiness of foods without increasing the amount of sodium chloride intake have conventionally been investigated.

However, currently proposed addition of potassium chloride, spices (pepper, ginger, etc.), flavoring ingredients, bitter substances (amino acids), and the like has the drawbacks of imparting bitterness, harshness, and unpleasant off-flavors such as odor, in addition to enhancing saltiness. Therefore, there has been a strong demand for a new technique for enhancing saltiness that does not have such drawbacks. In addition, from the aspect of enhancing the overall taste of foods and the like, a new technique for enhancing umami has also been desired.

Naringenin has also been used as a medicine, and under such circumstances, improvement of the taste of foods by using flavonoids including naringenin has been studied in the food industry (Patent Literatures 1 to 5). Patent Literature 1 relates to a "method for improving the flavor of food and drink, pharmaceutical product, or oral care product, including a step of adding γ-aminobutyric acid and naringenin". It describes the enhancement of umami, which is an effect of the combined use of naringenin and γ-aminobutyric acid, and does not describe the effect of naringenin alone. Furthermore, enhancement of saltiness is not considered. Patent Literature 2 relates to a "liquid seasoning containing (A) sodium, (B) flavonoids having a specific structure including naringenin, and (C) ethanol". It describes that the liquid seasoning suppresses color changes in foods, suppresses bitterness, astringency, and harshness derived from the substances, has a good flavor, and enhances the persistence of saltiness, which are the effects afforded by the three components (A), (B), and (C) together, and does not describe the effect of naringenin alone. Patent Literature 3 relates to "miso containing flavonoids" but does not disclose miso containing naringenin itself. It also describes that flavonoids containing flavonoid glycosides, rather than aglycones such as naringenin, are preferred in terms of solubility. It describes that coloring is suppressed and a miso with a good flavor is obtained, but does not describe enhancement of saltiness or umami. Patent Literature 4 relates to an "agent for improving aroma intensity and/or aroma quality of a flavoring composition, which contains γ-aminobutyric acid and naringenin" and does not describe enhancement of saltiness or umami. Patent Literature 5 relates to a "method for enhancing the sweetness of a sweetness modifier, including a step of adding an olfactory effective amount of naringenin, a salt thereof, or a combination of these" and does not describe enhancement of saltiness or umami.

### [Citation List]

### [Patent Literature]

[Patent Literature 1]
   WO 2013/008875
[Patent Literature 2]
   WO 2007/055426
[Patent Literature 3]
   WO 2009/096042
[Patent Literature 4]
   WO 2013/122226
[Patent Literature 5]
   JP-A-2015-188450

### [Summary of Invention]

### [Technical Problem]

There is a continuing need for a new technique that is different from conventional ones and aims to improve the decrease in satisfaction due to the insufficient saltiness in reduced-salt foods and the like, as well as a new technique for enhancing umami from the aspect of enhancing the overall taste of foods and the like.

### [Solution to Problem]

As described above, examination has been conducted also in the food field to improve taste by using flavonoids such as naringenin and the like, but no examination has been conducted for the effects of flavonoids per se such as naringenin and the like on enhancing saltiness and umami.

The present inventors have conducted intensive studies of the effects of naringenin and structurally similar flavonoid components (luteolin, pinocembrin, liquiritigenin, apigenin, and acacetin) on the improvement of the tastes of foods, and have unexpectedly found that the above-mentioned flavonoid components such as naringenin and the like have the effect of enhancing saltiness and umami of foods, and completed the present invention contributing to the improvement of the tastes of foods. Such effects of the above-mentioned flavonoid components such as naringenin and the like are not limited to foods, but contribute to the improvement of the tastes of all oral ingestion products including, for example, pharmaceutical products.

The present invention is described below with specific embodiments, but the present invention is not limited thereto.
[1] An agent for enhancing saltiness and/or umami of an oral ingestion product comprising a component imparting saltiness and/or a component imparting umami, the agent comprising one or two or more flavonoid components selected from naringenin, luteolin, pinocembrin, liquiritigenin, apigenin, and acacetin, or salts thereof, as an active ingredient.
[2] The agent of the above-mentioned [1], wherein the component imparting saltiness is one or two or more components selected from sodium chloride (NaCl), Na ion, and KCl.
[3] The agent of the above-mentioned [1], wherein the component imparting umami is one or two or more components selected from glutamate, aspartate, inosinate, and guanylate.
[4] The agent of any of the above-mentioned [1] to [3], wherein the oral ingestion product is a food or drink, or a pharmaceutical product.
[5] The agent of any of the above-mentioned [1] to [4], which is in the form of a composition containing one or two or more flavonoid components selected from naringenin, luteolin, pinocembrin, liquiritigenin, apigenin, and acacetin, or a salt thereof.
[6] The agent of the above-mentioned [5], which is in the form of a seasoning containing one or two or more flavonoid components selected from naringenin, luteolin, pinocembrine, liquiritigenin, apigenin, and acacetin, or a salt thereof.
[7] The agent of any of the above-mentioned [1] to [6], which is added to an oral ingestion product such that the content of one or two or more flavonoid components selected from naringenin, luteolin, pinocembrin, liquiritigenin, apigenin, and acacetin, or a salt thereof in the oral ingestion product is 0.01 weight ppm to 100 weight ppm (preferably, 0.1 weight ppm to 100 weight ppm) as flavonoid component when orally ingested.
[8] The agent of any of the above-mentioned [1] to [7], wherein the flavonoid component is naringenin.
[9] A method for producing an oral ingestion product having enhanced saltiness and/or umami, comprising adding the agent of any of the above-mentioned [1] to [8] to an oral ingestion product containing a component imparting saltiness and/or a component imparting umami.
[10] An oral ingestion product containing a component imparting saltiness and/or a component imparting umami (excluding oral ingestion products containing γ-aminobutyric acid), further comprising the agent of any of the above-mentioned [1] to [9].
[11] A method for enhancing the saltiness and/or umami of an oral ingestion product containing a component imparting saltiness and/or a component imparting umami, comprising adding one or two or more flavonoid components selected from naringenin, luteolin, pinocembrin, liquiritigenin, apigenin, and acacetin, or a salt thereof to the oral ingestion product.
[12] The method of the above-mentioned [11], wherein the flavonoid component is naringenin.
[13] One or two or more flavonoid components selected from naringenin, luteolin, pinocembrin, liquiritigenin, apigenin, and acacetin, or a salt thereof, which is used to enhance the saltiness and/or umami of an oral ingestion product containing a component imparting saltiness and/or a component imparting umami.
[14] The flavonoid component or salt thereof of the above-mentioned [13], wherein the flavonoid component is naringenin.

### [Advantageous Effects of Invention]

The present invention provides, as its embodiments, an agent for enhancing saltiness and/or umami, containing one or two or more flavonoid components selected from naringenin, luteolin, pinocembrin, liquiritigenin, apigenin, and acacetin, or salts thereof, as active ingredients, which agent can enhance the overall taste of an oral ingestion product by enhancing the saltiness and/or umami of the oral ingestion product containing a component imparting saltiness and/or a component imparting umami, without imparting an off-flavor; and the like.

### [Description of Embodiments]

One embodiment of the present invention relates to an agent for enhancing saltiness and/or umami of an oral ingestion product containing a component imparting saltiness and/or a component imparting umami, the agent containing one or two or more flavonoid components selected from naringenin, luteolin, pinocembrin, liquiritigenin, apigenin, and acacetin, or salts thereof, as an active ingredient (the first embodiment)".

It is described in detail below.

The above-mentioned agent for enhancing saltiness and/or umami (hereinafter also referred to as "the present enhancer") is characterized by containing one or two or more flavonoid components selected from naringenin, luteolin, pinocembrin, liquiritigenin, apigenin, and acacetin (hereinafter also referred to as "the present flavonoid component") or a salt thereof as an active ingredient.

In the present enhancer, any one component selected from naringenin, luteolin, pinocembrin, liquiritigenin, apigenin, and acacetin may be used alone as the flavonoid component, or two or more (preferably 2 to 3) different components may be combined and used as the flavonoid component. All forms of the flavonoid component are included within the scope of the present enhancer.

Naringenin is a naturally occurring compound having the following structure (CAS number: 480-41-1; IUPAC name: 5,7-dihydroxy-2-(4-hydroxyphenyl)chroman-4-one), and is contained in citrus fruits, onion, tomato, and the like. It is slightly sweet at about 1000 weight ppm and has no bitterness.

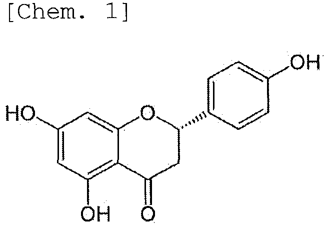

Luteolin is a naturally occurring compound having the following structure (CAS number: 491-70-3; IUPAC name: 2-(3,4-dihydroxyphenyl)-5,7-dihydroxy-4-chromenone), and is contained in celery, broccoli, green pepper, and the like.

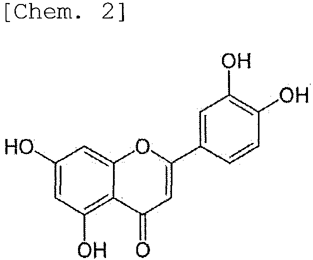

Pinocembrin is a naturally occurring compound having the following structure (CAS number: 68745-38-0(±), 480-39-7(S)-(+), 206660-42-6(R)-(-); IUPAC name: 5,7-dihydroxy-2-phenyl-2,3-dihydro-4H-chromene-4-one), and is contained in damiana, honey, propolis, and the like.

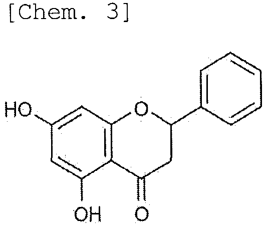

Liquiritigenin is a naturally occurring compound having the following structure (CAS number: 578-86-9; IUPAC name: (2S)-4',7-dihydroxyflavan-4-one), and is contained in licorice and the like.

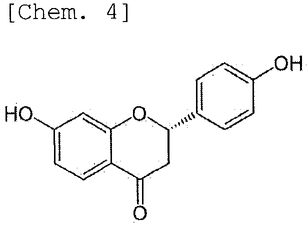

Apigenin is a naturally occurring compound having the following structure (CAS number: 520-36-5; IUPAC name: 5,7-dihydroxy-2-(4-hydroxyphenyl)-4H-1-benzopyran-4-one), and is contained in parsley, celery, chamomile, and the like.

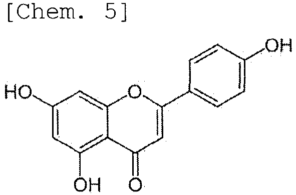

Acacetin is a naturally occurring compound having the following structure (CAS Number: 480-44-4; IUPAC name: 5,7-dihydroxy-4-methoxyflavone), and is contained in black locust, damiana, and the like.

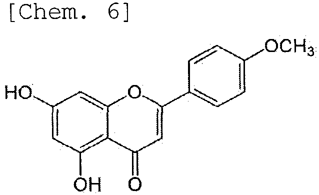

In the present enhancer, the present flavonoid component can be used in the form of a salt thereof. The above-mentioned salt is not particularly limited as long as it is an edible salt and does not impair the purpose of the present invention. For example, salts with alkali metals such as sodium, potassium and the like; salts with alkaline earth metals such as calcium, magnesium and the like; ammonium salt; aluminum salt; zinc salt; salts with organic amines such as triethylamine, ethanolamine, morpholine, pyrrolidine, piperidine, piperazine, dicyclohexylamine and the like; salts with basic amino acids such as arginine, lysine and the like can be mentioned. Any one kind of these salts may be used alone, or two or more kinds may be used in combination.

The present flavonoid component may have stereoisomers, and taking naringenin as an example, there are stereoisomers (2S-naringenin or 2R-naringenin) and stereoisomeric mixtures. All of these stereoisomers and mixtures thereof are included in the present flavonoid component in the present enhancer. In addition, this present flavonoid component may be a hydrate (hydrate salt) and examples of such hydrate include 1 to 6 hydrates and the like. The hydrate is also included in the flavonoid component in the present enhancer.

The present flavonoid component or a salt thereof may be a synthetic product, an isolated and purified form, or a plant extract, as long as it is suitable for oral ingestion. Commercially available products can also be used.

The form of the present enhancer is not particularly limited and, for example, solid (including powder, granular and the like), liquid (including slurry and the like), gel, paste and the like can be mentioned.

Examples of the base when the present enhancer is in a liquid form include water, ethanol, glycerol, propylene glycol, various animal or vegetable oils and the like.

Examples of the base when the present enhancer is in a solid form include starch, dextrin, cyclodextrin, various saccharides such as sucrose, glucose and the like, protein, peptide, sodium chloride, solid fat, silicon dioxide, and a mixture thereof, yeast fungus, various powder extracts and the like.

The present enhancer may consist of only the above-mentioned active ingredient ("present flavonoid component alone"), but may also be in the form of a composition that further contains, in addition to the active ingredient, a base conventionally used in the food field and pharmaceutical field ("present flavonoid component-containing composition").

When the present enhancer is used in the form of a "present flavonoid component-containing composition", it may further contain, for example, excipient, pH adjuster, antioxidant, thickening stabilizer, sweetener, acidulant, spice, colorant and the like as long as the purpose of the present invention is not impaired. For example, it may be prepared as a seasoning.

The content of the present flavonoid component or a salt thereof in the present enhancer is preferably 0.0000001 wt% or more, more preferably 0.00001 wt% or more, particularly preferably 0.001 wt% or more, as the present flavonoid component, with respect to the total amount of the present enhancer. The content is preferably 99.9 wt% or less, more preferably 99 wt% or less, particularly preferably 95 wt% or less, with respect to the present enhancer.

For example, the present flavonoid component or a salt thereof can be contained within the range of 0.01 weight ppm to 500000 weight ppm, preferably 0.01 weight ppm to 100000 weight ppm, more preferably 0.01 weight ppm to 10000 weight ppm, further preferably 0.01 weight ppm to 1000 weight ppm, particularly preferably 0.01 weight ppm to 100 weight ppm, relative to the total amount of the present enhancer as the present flavonoid component.

The present enhancer can be produced by a method known per se (e.g., a method conventionally used in the flavor field). The present enhancer may be subjected to, for example, a concentration treatment, a drying treatment, a decolorization treatment and the like alone or combination.

The present enhancer is used to enhance a saltiness and/or umami of an oral ingestion product. The "oral ingestion product" in which the present enhancer is used is a concept widely encompassing those that can be ingested orally, and also includes, in addition to food and drink (a general term for food products and drinks), seasoning, food additive, oral medicine (including quasi-drugs such as oral care products), supplement, and the like. The oral ingestion product in which the present enhancer is used is not particularly limited as long as it is desired to have enhanced saltiness and/or umami, and is preferably a food or drink, or seasoning. The food or drink in which the present enhancer is used may be provided, for example, as a food with health claims, a food for specified health uses, a food with nutrient function claims, a dietary supplement, a nutritional supplementary food, a health supplementary food, a food for medical use, a medical food or the like.

The oral ingestion product in which the present enhancer is used may be provided (sold, distributed) in a form suitable for oral ingestion, or may be provided in a form that requires a predetermined treatment or cooking to be made suitable for oral ingestion (for example, as a food ingredient such as vegetable, fish, and meat used in cooking for eating). For example, the oral ingestion product in which the present enhancer is used may be provided as a concentrate that requires dilution with water or the like to be made suitable for use in oral ingestion product. In this case, the content, concentration, and amount of each component to be added when used in the oral ingestion product may be appropriately adjusted according to the dilution ratio and the like.

The present enhancer is preferably used to enhance the saltiness and/or umami of an oral ingestion product containing a component imparting saltiness and/or a component imparting umami. As used herein, the "oral ingestion product containing a component imparting saltiness and/or a component imparting umami" means an oral ingestion product containing "a component imparting saltiness" and/or "a component imparting umami" as an essential component.

The "enhancement of a saltiness" afforded by the present enhancer means that the saltiness is strongly felt as if the concentration of "a component imparting a saltiness" was increased. The presence or absence and the degree of saltiness can be evaluated by a sensory evaluation by an expert panel (e.g., sensory evaluation etc. shown in Examples described later).

The "enhancement of umami" afforded by the present enhancer means that the umami is strongly felt as if the concentration of "a component imparting umami" was increased. The presence or absence and the degree of umami can be evaluated by a sensory evaluation by an expert panel (e.g., sensory evaluation etc. shown in Examples described later).

The "component imparting saltiness" in the "oral ingestion product containing a component imparting saltiness and/or a component imparting umami" in which the present enhancer is used refers to a component that has a saltiness by itself and can present a saltiness in the mouth. Examples include sodium chloride (NaCl), sodium ion, and KCl. Here, the sodium ion may be contained in the form of a salt in the oral ingestion product. As the salt form, inorganic sodium salt, organic acid sodium salt, amino acid sodium salt, nucleic acid sodium salt, and the like can be used. Specific examples include sodium chloride, sodium glutamate, sodium aspartate, sodium gluconate, sodium succinate, sodium inosinate, sodium guanylate, and mixtures of two or more of these.

The "oral ingestion product" in which the present enhancer is used may contain one component selected from sodium chloride, sodium ion, and KCl as a saltiness component, or may contain two or more components in combination.

The "component imparting umami" in the "oral ingestion product containing a component imparting saltiness and/or a component imparting umami" in which the present enhancer is used refers to a component that has a umami by itself and can present a umami in the mouth. Examples include glutamates (e.g., sodium glutamate, calcium glutamate, potassium glutamate, etc.), aspartates (e.g., sodium aspartate, potassium aspartate, arginine aspartic acid, etc.), inosinates (e.g., sodium inosinate, calcium inosinate, potassium inosinate, etc.), guanylates (e.g., sodium guanylate, calcium guanylate, etc.) and the like.

The "oral ingestion product" in which the present enhancer is used may contain one component selected from the above-mentioned glutamate, aspartate, inosinate, and guanylate as a umami component, or may contain two or more components in combination.

In one form, the content of each of the "component imparting saltiness" and the "component imparting umami" in the "oral ingestion product containing a component imparting saltiness and/or a component imparting umami" to which the present enhancer is added is, for example, 0.0000001 wt% or more, preferably 0.0000005 wt% or more, more preferably 0.000001 wt% or more, further preferably 0.000005 wt% or more; or, for example, 0.0000001 to 100 wt%, preferably 0.0000005 to 100 wt%, more preferably 0.000001 to 100 wt%, further preferably 0.000005 to 100 wt%, particularly preferably 0.00001 to 95 wt%, especially preferably 0.0001 to 90 wt%, more particularly preferably 0.001 to 70 wt%, further more preferably 0.01 to 50 wt%, still more preferably 0.1 to 30 wt%, yet more preferably 0.1 to 10 wt%, further more preferably 0.1 to 5 wt%.

In the present invention, when the oral ingestion product containing a component imparting saltiness and/or a component imparting umami is a food or drink that is eating after dilution and the like, the contents of the above-mentioned "component imparting saltiness" and "component imparting umami" respectively refer to the contents (concentrations) at the time of eating.

The oral ingestion product containing a component imparting saltiness and/or a component imparting umami, to which the present enhancer is added, is preferably one desired to have enhanced saltiness and/or umami.

Taking a food or drink as an example, a sodium chloride-containing food is one example. While the sodium chloride-containing food to which the present enhancer can be added is not particularly limited, one desired to have an enhanced saltiness and/or umami is preferred. For example, soup (including dried soup) such as corn soup, consomme soup (e.g., chicken, pork, beef etc.), potage, soup with egg, soup with Wakame seaweed, soup with shark fin, Chinese-style soup, curry flavor soup, Ramen noodle soup, Japanese-style clear soup, Miso soup and the like; meat processed food such as ham, sausage, Gyoza dumpling, Shumai dumpling, hamburg steak, deep-fried food, pork cutlet and the like; marine processed food such as steamed fish paste cake, tube shaped fish paste cake and the like; dairy product such as butter and the like; rice processed food such as Chinese fried rice and the like; seasoning such as savory seasoning, flavor seasoning, menu seasoning, mayonnaise, dressing, sauce (e.g., demi-glace sauce, Japanese Worcestershire-style sauce, white sauce, cheese sauce, carbonara sauce etc.) and the like; other processed food such as noodles, gratin, croquette, pickles and the like; frozen food (frozen products of the aforementioned foods (e.g., Gyoza dumpling, Shumai dumpling, Chinese fried rice, hamburg steak, deep-fried food, gratin, pork cutlet, croquette etc.), etc.) and the like can be mentioned.

The above-mentioned "savory seasoning" refers to a seasoning produced from a natural substance as a material and by a method such as extraction, decomposition, heating, fermentation, and the like. Concrete examples thereof include various meat extracts such as chicken meat extract, beef meat extract, pork meat extract, sheep meat extract and the like; various bone extracts such as chicken bone extract, beef bone extract, pork bone extract and the like; various seafood extracts such as bonito extract, mackerel extract, croaker extract, scallop extract, crab extract, shrimp extract, dried sardine extract, dried adductor extract and the like; various dried fish extracts such as dried bonito extract, dried mackerel extract, dried bullet mackerel extract and the like; various vegetable extracts such as onion extract, Chinese cabbage extract, celery extract and the like; various seaweed extracts such as kelp extract and the like; various spice extracts such as garlic extract, chili extract, pepper extract, cacao extract and the like; yeast extracts; various protein hydrolysate; various fermentation seasonings such as soy sauce, fish sauce, shrimp sauce, Miso and the like, and the like and mixtures thereof, processed product (e.g., soy sauce processed product such as Japanese noodle soup base, citrus seasoned soy sauce and the like), and the like.

The above-mentioned "flavor seasoning" refers to a seasoning used to impart aroma, flavor and taste of a flavor material to a food, and can be produced by, for example, adding sugar, sodium chloride and the like to a savory seasoning, or the like. Specific examples of the flavor seasoning include various meat flavor seasonings such as chicken flavor seasoning, beef flavor seasoning, pork flavor seasoning and the like; various seafood flavor seasonings such as bonito flavor seasoning, dried sardine flavor seasoning, dried adductor flavor seasoning, crustacean flavor seasoning and the like; various spicy vegetable flavor seasonings; kelp flavor seasoning and the like.

The above-mentioned "menu seasoning" refers to a seasoning suitable for cooking a particular menu (Chinese-style menu etc.). Concrete examples thereof include Chinese-style combined seasoning, combined seasoning, generic paste seasoning, seasoning for hot pot, seasoning mix for cooked rice mixed with ingredients, seasoning mix for Chinese fried rice, spice mix and the like.

The sodium chloride-containing food to which the present enhancer can be added may be provided (sold, distributed) in a form suitable for eating, or may be provided in a form that requires a predetermined processing or cooking to have a form suitable for eating. For example, the sodium chloride-containing food to which the enhancer of the present invention can be added may be provided (sold, distributed) as a concentrate or the like that requires diluting with water etc. to become a form suitable for eating.

The method and conditions for adding the present enhancer to an oral ingestion product containing a component imparting saltiness and/or a component imparting umami are not particularly limited, and can be appropriately set by a person skilled in the art according to the form of the present enhancer and the type of the oral ingestion product containing a component imparting saltiness and/or a component imparting umami.

The timing of addition of the present enhancer to an oral ingestion product containing a component imparting saltiness and/or a component imparting umami is not particularly limited, and it may be added at any time. For example, during production of food or drink (during cooking), after completion of food or drink (e.g., immediately before eating food or drink, during eating food or drink, etc.) and the like can be mentioned. Alternatively, the present enhancer may be added in advance to raw materials prior to the production of an oral ingestion product containing a component imparting saltiness and/or a component imparting umami.

For effective enhancement of saltiness and/or umami, the present enhancer is added to an oral ingestion product containing a component imparting saltiness and/or a component imparting umami such that the content of the present flavonoid component or a salt thereof in the oral ingestion product is preferably 0.001 weight ppb or more, more preferably 0.01 weight ppb or more, further preferably 0.05 weight ppb or more, particularly preferably 0.1 weight ppb or more; or preferably 1000000 weight ppb or less, more preferably 500000 weight ppb or less, further preferably 100000 weight ppb or less, particularly preferably 50000 weight ppb or less, especially preferably 10000 weight ppb or less; or preferably 0.00001 to 10000000 weight ppb, more preferably 0.0001 to 5000000 weight ppb, further preferably 0.0001 to 1000000 weight ppb, further more preferably 0.0001 to 500000 weight ppb, particularly preferably 0.005 to 500000 weight ppb, especially preferably 0.05 to 300000 weight ppb, more particularly preferably 0.1 to 100000 weight ppb, further more preferably 1 to 100000 weight ppb, still more preferably 10 to 100000 weight ppb, further still more preferably 100 to 100000 weight ppb, even more preferably 1000 to 50000 weight ppb, as the present flavonoid component when orally ingested. A specific amount to be added can be appropriately determined by a person skilled in the art depending on the oral ingestion product of interest.

The oral ingestion product containing a component imparting saltiness and/or a component imparting umami, in which the present enhancer is used, may contain other taste components (e.g., component imparting sweetness, component imparting sourness, component imparting bitter taste, etc.) in addition to the "component imparting saltiness" and "component imparting umami".

Another embodiment of the present invention relates to "a method for producing an oral ingestion product having enhanced saltiness and/or umami, comprising adding the present enhancer to an oral ingestion product containing a component imparting saltiness and/or a component imparting umami (the second embodiment)".

In this embodiment, the technical meanings of terms such as "a component imparting saltiness", "a component imparting umami", "an oral ingestion product containing a component imparting saltiness and/or a component imparting umami", "the present enhancer", "enhanced saltiness and/or umami", and the like and preferred concrete forms thereof can be referred to the above-mentioned description of "the present enhancer" in the first embodiment.

The "production method of an oral ingestion product having enhanced saltiness and/or umami" in this embodiment includes adding the present enhancer to an oral ingestion product containing a component imparting saltiness and/or a component imparting umami. The method, conditions, and timing of addition here are not particularly limited, and a person skilled in the art can appropriately set them according to the form of the present enhancer and the type of the oral ingestion product containing a component imparting saltiness and/or a component imparting umami, and produce an oral ingestion product having enhanced saltiness and/or umami.

A still another embodiment of the present invention relates to "an oral ingestion product containing a component imparting saltiness and/or a component imparting umami (excluding oral ingestion products containing γ-aminobutyric acid), further comprising the present enhancer (the third embodiment)".

In this embodiment, the technical meanings of terms such as "the present enhancer", "a component imparting saltiness", "a component imparting umami", "an oral ingestion product containing a component imparting saltiness and/or a component imparting umami", and the like and preferred concrete forms thereof can be referred to the above-mentioned description of "the present enhancer" in the first embodiment.

The oral ingestion product in this embodiment can be produced by adding the present enhancer to an oral ingestion product containing a component imparting saltiness and/or a component imparting umami and not containing γ-aminobutyric acid, according to the production method of the above-mentioned second embodiment.

Another embodiment of the present invention relates to "a method for enhancing the saltiness and/or umami of an oral ingestion product containing a component imparting saltiness and/or a component imparting umami, comprising adding one or two or more flavonoid components (present flavonoid component) selected from naringenin, luteolin, pinocembrin, liquiritigenin, apigenin, and acacetin, or a salt thereof to the oral ingestion product (the fourth embodiment)".

In this embodiment, the technical meanings of terms such as "a component imparting saltiness", "a component imparting umami", "an oral ingestion product containing a component imparting saltiness and/or a component imparting umami", "enhanced saltiness and/or umami", "present flavonoid component or a salt thereof", "adding the present flavonoid component or a salt thereof", and the like and preferred concrete forms thereof can be referred to the above-mentioned description of "the present enhancer" in the first embodiment.

The enhancement method of this embodiment is performed by adding the present flavonoid component or a salt thereof to "an oral ingestion product containing a component imparting saltiness and/or a component imparting umami". The amount of the present flavonoid component or a salt thereof to be added to the oral ingestion product can be appropriately determined by a person skilled in the art with reference to the explanation of "the content of the present flavonoid component or a salt thereof in the oral ingestion product when orally ingested" in the first embodiment. In addition, the method in this embodiment includes adding the present flavonoid component or a salt thereof to "an oral ingestion product containing a component imparting saltiness and/or a component imparting umami". The method, conditions, and timing of addition here are not particularly limited, and a person skilled in the art can appropriately set them according to the form of the present flavonoid component or a salt thereof as an enhancer and the type of the oral ingestion product containing a component imparting saltiness and/or a component imparting umami. When added, the form of the present flavonoid component or a salt thereof as an enhancer is not particularly limited, and a person skilled in the art can appropriately add same in the form of "the present flavonoid component alone" or "the present flavonoid component-containing composition" with reference to the form described above for the first embodiment.

Other embodiment of the present invention relates to "one or two or more flavonoid components (present flavonoid component) selected from naringenin, luteolin, pinocembrin, liquiritigenin, apigenin, and acacetin, or a salt thereof, which is used to enhance the saltiness and/or umami of an oral ingestion product containing a component imparting saltiness and/or a component imparting umami (the fifth embodiment)".

In this embodiment, the technical meanings of terms such as "a component imparting saltiness", "a component imparting umami", "an oral ingestion product containing a component imparting saltiness and/or a component imparting umami", "enhanced saltiness and/or umami", "present flavonoid component or a salt thereof", and the like and preferred concrete forms thereof can be referred to the above-mentioned description of "the present enhancer" in the first embodiment.

### [Example]

The present invention is explained in more detail below with reference to Examples, but the present invention is not limited to these Examples.

### Example 1: Enhancement of saltiness (1)

### [Confirmation of effect of adding naringenin to saltiness aqueous solution]

### 1. Preparation of evaluation samples

### (1) Preparation of evaluation base solution

As shown in Table 1: NaCl aqueous solution (weight g/100 g), various NaCl aqueous solutions (saline solutions) were prepared. Filtered water at ordinary temperature was used for preparation.

**[Table 1]**

| starting material | manufacturer | salt value (%) | NC (0.8%) | | PC Low (0.9%) | | PC High (1. 0%) | |
|---|---|---|---|---|---|---|---|---|
| | | | blend amount (g) | salt value | blend amount (g) | salt value | blend amount (g) | salt value |
| sodium chloride | Naikai Salt Industries Co., Ltd. | 100.00 | 0.80 | 0.80 | 0.90 | 0.90 | 1.00 | 1.00 |
| water (ordinary temperature) | filtered water | 0.00 | 99.20 | 0.00 | 99.10 | 0.00 | 99.00 | 0.00 |
| | | | total | 0.80 | total | 0.90 | total | 1.00 |

### (2) Addition of naringenin

Water, naringenin (Penta Manufacturing), or naringin (IKEDA CORPORATION) were weighed and added to the prepared 0.8% aqueous solution at various concentrations shown in Table 2: Prepared sensory evaluation samples.

**[Table 2]**

| | test plot | added solution | NaCl (%) | naringenin (ppm) | naringin (ppm) |
|---|---|---|---|---|---|
| 1 | NC | salt water | 0.8 | 0 | 0 |
| 2 | PC | salt water | 0.9 | 0 | 0 |
| 3 | water+Naringenin 30 ppm | water | 0 | 30 | 0 |
| 4 | water+Naringin 30 ppm | water | 0 | 0 | 30 |
| 5 | NC+Naringenin 1 ppm | salt water | 0.8 | 1 | 0 |
| 6 | NC+Naringenin 5 ppm | salt water | 0.8 | 5 | 0 |
| 7 | NC+Naringenin 10 ppm | salt water | 0.8 | 10 | 0 |
| 8 | NC+Naringenin 30 ppm | salt water | 0.8 | 30 | 0 |
| 9 | NC+Naringin 5 ppm | salt water | 0.8 | 0 | 5 |
| 10 | NC+Naringin 10 ppm | salt water | 0.8 | 0 | 10 |
| 11 | NC+Naringin 30 ppm | salt water | 0.8 | 0 | 30 |

### 2. Evaluation of saltiness enhancement function by sensory evaluation

The saltiness intensity of each sample was blindly evaluated using a scoring system in 0.1 point increments, with the saltiness intensity of NC in test plot 1 as 1.00 point and the saltiness intensity of PC in test plot 2 as 5.5 points. The evaluation was performed by a trained panel of three, and the average score of each panel was used. Samples with a saltiness intensity of 1.8 points or more (NaCl 0.84% or more) due to the addition were evaluated to "have a saltiness enhancement function". In addition, the presence or absence, and intensity of off-taste (bitterness and harsh taste other than saltiness) were also evaluated. The evaluation results are shown in Table 3: Sensory evaluation (saltiness intensity).

**[Table 3]**

| | test plot | added solution | saltiness intensity | off-taste | off-taste intensity |
|---|---|---|---|---|---|
| 1 | NC | salt water | 1 | none | - |
| 2 | PC | salt water | 5.5 | none | - |
| 3 | water+Naringenin 30 ppm | water | 0 | none | - |
| 4 | water+Naringin 30 ppm | water | 0 | slightly bitter | + |
| 5 | NC+Naringenin 1 ppm | salt water | 1.9 | none | - |
| 6 | NC+Naringenin 5 ppm | salt water | 2.4 | none | - |
| 7 | NC+Naringenin 10 ppm | salt water | 3.0 | none | - |
| 8 | NC+Naringenin 30 ppm | salt water | 2.9 | none | - |
| 9 | NC+Naringin 1 ppm | salt water | 1.8 | slightly bitter | + |
| 10 | NC+Naringin 10 ppm | salt water | 2.1 | moderately bitter | ++ |
| 11 | NC+Naringin 30 ppm | salt water | 2.2 | strongly bitter | +++ |

As is clear from the above-mentioned results, the addition of 1 ppm or more of naringenin showed a saltiness enhancement function on salt water, without imparting an off-taste. On the other hand, the addition of 1 ppm or more of naringin also showed a saltiness enhancement function, but the degree of enhancement was inferior to that of naringenin addition, and an unpleasant bitterness was simultaneously imparted.

From the above, it was clarified that naringenin affords a very superior saltiness enhancement effect due to the saltiness enhancement function.

### Example 2: Enhancement of umami (1)

### [Confirmation of effect of adding naringenin to umami solution]

### 1. Preparation of evaluation samples

### (1) Preparation of evaluation base solution

As shown in Table 4: Umami solution (weight g/100 g), an umami solution was prepared. Water at ordinary temperature was used for preparation.

**[Table 4]**

| starting material | manufacturer | purity (%) | NC1 (0.1%) | NC2 (0.2%) |
|---|---|---|---|---|
| | | | blend amount (g) | blend amount (g) |
| sodium glutamate | Ajinomoto Co., Inc. | 100.00 | 0.10 | - |
| sodium aspartate | Ajinomoto Co., Inc. | 100.00 | - | 0.20 |
| water (ordinary temperature) | filtered water | 0.00 | 99.90 | 99.80 |
| | | | total | total |

### (2) Addition of naringenin

Water, naringenin (Penta manufacturing) or naringin (IKEDA CORPORATION) were weighed and added to each of the prepared umami solutions or water at various concentrations shown in Table 5: Prepared sensory evaluation samples.

**[Table 5]**

| | test plot | added solution | sodium glutamate (ppm) | sodium aspartate (ppm) | Naringenin (ppm) |
|---|---|---|---|---|---|
| 1 | water | - | - | - | - |
| 2 | water+Naringenin 30 ppm | water | - | - | 30 |
| 3 | NC1 (MSG) | umami solution | 1000 | - | - |
| 4 | NC2(Asp-Na) | umami solution | - | 2000 | - |
| 5 | NC1+Naringenin 30 ppm | umami solution | 1000 | - | 30 |
| 6 | NC2+Naringenin 30 ppm | umami solution | - | 2000 | 30 |

### 2. Evaluation of umami enhancement function by sensory evaluation

The umami intensity of each sample was blindly evaluated using a scoring system in 0.01 point increments, with the umami intensity of water in test plot 1 as 0 point, the umami intensity of NC1 in test plot 3 as 0.30 points, and the umami intensity of NC2 in test plot 4 as 0.10 point. The evaluation was performed by a trained panel of three, and the average score of each panel was used. Samples whose umami intensity was enhanced by 1.5 times or more (sodium glutamate 0.45 points or more, sodium aspartate 0.15 points or more) due to the addition were evaluated to "have an umami enhancement function". In addition, the presence or absence, and intensity of off-taste (bitterness and harsh taste other than umami) were also evaluated. The evaluation results are shown in Table 6: Sensory evaluation (umami intensity).

**[Table 6]**

| | test plot | umami intensity (points) | degree of umami enhancement (times) | off-taste | off-taste intensity |
|---|---|---|---|---|---|
| 1 | water | 0 | - | - | - |
| 3 | NC1 (MSG) | 0.30 | - | none | - |
| 4 | NC2 (Asp-Na) | 0.10 | - | none | - |
| 2 | water+Naringenin 30 ppm | 0.00 | 0 | none | - |
| 3 | water+Naringin 30 ppm | 0.00 | 0 | slightly bitter | + |
| 5 | NC1+Naringenin 30 ppm | 0.47 | 1.6 | none | - |
| 6 | NC2+Naringenin 30 ppm | 0.20 | 2.0 | none | - |
| 7 | NC1+Naringin 30 ppm | 0.35 | 1.2 | slightly bitter | + |
| 8 | NC2+Naringin 30 ppm | 0.12 | 1.2 | slightly bitter | + |

As is clear from the above-mentioned results, the addition of naringenin showed an umami enhancement function on umami solution, without imparting an off-taste. On the other hand, the addition of naringin imparted a bitter taste, and no enhancement of the umami intensity was observed.

From the above, it was clarified that naringenin affords a very superior umami enhancement effect due to the umami enhancement function.

### (Summary of evaluation results)

It was clarified that an enhancer containing naringenin or a salt thereof as an active ingredient can enhance the saltiness and/or umami of an oral ingestion product containing a component imparting saltiness and/or a component imparting umami, without imparting "an off-taste or off-flavor such as bitterness, harsh taste, and the like", and contributes to the improvement of the overall tastes of oral ingestion products.

### Example 3: Enhancement of saltiness (2)

### [Confirmation of effect of adding naringenin-related flavonoid components to saltiness aqueous solution]

### 1. Preparation of evaluation samples

### (1) Flavonoid components evaluated

1) The following flavonoid components were used as naringenin-related flavonoid components:
   Luteolin (manufactured by Salus Nutra Inc.), pinocembrin (manufactured by Xi'an Demeter Biotech Co., Ltd.), liquiritigenin (manufactured by Xi'an Julong Import and Export Co., Ltd.), apigenin (manufactured by Xi'an Best Bio-Tech Co., Ltd.), and acacetin (manufactured by Xi'an Reain Biotechnology Co., Ltd.)
2) The following compound was used as control component: Quercetin (manufactured by Medience Corporation) (structure shown below)

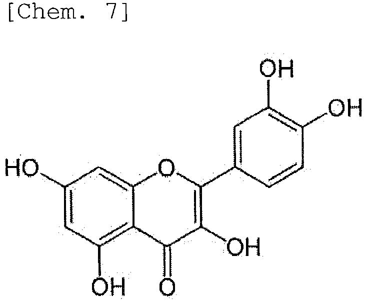

### (2) Preparation of evaluation base solutions

As shown in Table 7: NaCl aqueous solution (weight g/100 g), various NaCl aqueous solutions (saline solutions) were prepared. Water at ordinary temperature was used for preparation.

**[Table 7]**

| starting material | manufacturer | NC (0.8%) | | PC1 Low (0.9%) | | PC2 High (1.0%) | |
|---|---|---|---|---|---|---|---|
| | | blend amount (g) | salt value | blend amount (g) | salt value | blend amount (g) | salt value |
| sodium chloride | Naikai Salt Industries Co., Ltd. | 0.80 | 0.80 | 0.90 | 0.90 | 1.00 | 1.00 |
| water (ordinary temperature) | filtered water | 99.20 | 0.00 | 99.10 | 0.00 | 99.00 | 0.00 |
| salt value (%) | | total | 0.80 | total | 0.90 | total | 1.00 |

### (3) Addition of naringenin-related flavonoid component, etc.

Each naringenin-related flavonoid component dissolved in propylene glycol (SIGMA) at 10000 ppm was added to the prepared 0.8% salt water solution such that the concentration at the time of eating (corresponding to the content of the present flavonoid component or a salt thereof in the oral ingestion product at the time of oral ingestion, as a flavonoid component relative to the oral ingestion product) was 30 ppm (Table 8).

**[Table 8]**

| test plot | sample | NaCl (%) | flavonoid concentration (ppm) |
|---|---|---|---|
| 1 | NC | 0.8 | 0 |
| 2 | PC1 Low | 0.9 | 0 |
| 3 | PC2 High | 1.0 | 0 |
| 4 | NC+Quercetin | 0.8 | 30 |
| 5 | NC+Luteolin | 0.8 | 30 |
| 6 | NC+Pinocembrin | 0.8 | 30 |
| 7 | NC+Liquiritigenin | 0.8 | 30 |
| 8 | NC+Apigenin | 0.8 | 30 |
| 9 | NC+Acacetin | 0.8 | 30 |

### 2. Evaluation of saltiness enhancement function by sensory evaluation

The saltiness intensity of each sample was blindly evaluated using a scoring system in 0.5 point increments, with the saltiness intensity of NC in test plot 1 as 1.0 point, the saltiness intensity of PC1 as 2.0 points, and the saltiness intensity of PC2 as 3.0 points. The evaluation was performed by a trained panel of three, and the average score was used. Since the saltiness intensity of 2.0 points of PC1 is evaluated to be clearly stronger in saltiness than NC, samples with a saltiness intensity of 1.5 points or more (NaCl 0.85% or more) were evaluated to have a saltiness enhancement function. In addition, the presence or absence, and intensity of off-taste (bitterness and harsh taste other than saltiness) were also evaluated. The evaluation results are shown in Table 9: Sensory evaluation (saltiness intensity).

**[Table 9]**

| test plot | sample | NaCl (%) | flavonoid concentration (ppm) | saltiness intensity | off-taste | off-taste intensity |
|---|---|---|---|---|---|---|
| 1 | NC | 0.8 | 0 | 1.0 | none | - |
| 2 | PC1 Low | 0.9 | 0 | 2.0 | none | - |
| 3 | PC2 High | 1.0 | 0 | 3.0 | none | - |
| 4 | NC+Quercetin | 0.8 | 30 | 1.3 | bitter | + |
| 5 | NC+Luteolin | | | 2.0 | none | - |
| 6 | NC+Pinocembrin | | | 2.0 | none | - |
| 7 | NC+ Liquiritigenin | | | 3.5 | none | - |
| 8 | NC+Apigenin | | | 3.5 | none | - |
| 9 | NC+Acacetin | | | 3.5 | none | - |

As in the above-mentioned results, the addition of five flavonoids excluding quercetin was found to show an effect of enhancing the saltiness intensity of salt water, without imparting an off-taste. On the other hand, the addition of quercetin did not show a saltiness enhancement effect, and bitterness was simultaneously imparted.

### Example 4: Enhancement of umami (2)

### [Confirmation of effect of adding naringenin-related flavonoid components to umami solution]

### 1. Preparation of evaluation samples

### (1) Flavonoid components evaluated

1) The five naringenin-related flavonoid components evaluated in Example 3 were used.
2) Quercetin was used as the control component.

### (2) Preparation of evaluation base solution

Umami solutions were prepared as shown in Table 10: Umami solution (weight g/100 g). Water at ordinary temperature was used for preparation.

**[Table 10]**

| starting material | manufacturer | NC1 (0.1%) |
|---|---|---|
| | | blend amount (g) |
| sodium glutamate | Ajinomoto Co., Inc. | 0.1 |
| water (ordinary temperature) | filtered water | 99.9 |

### (3) Addition of naringenin-related flavonoid component, etc.

Each naringenin-related flavonoid component and the like dissolved in propylene glycol (SIGMA) at 10000 ppm was added to the prepared 0.1% umami aqueous solution such that the concentration at the time of eating was 30 ppm (Table 11).

**[Table 11]**

| test plot | sample | sodium glutamate (%) | flavonoid concentration (ppm) |
|---|---|---|---|
| 1 | water | 0 | 0 |
| 2 | NC | 0.1 | 0 |
| 3 | NC+Quercetin | | 30 |
| 4 | NC+Luteolin | | |
| 5 | NC+Pinocembrin | | |
| 6 | NC+Liquiritigenin | | |
| 7 | NC+Apigenin | | |
| 8 | NC+Acacetin | | |

### 2. Evaluation of umami enhancement function by sensory evaluation

The umami intensity of each sample was blindly evaluated using a scoring system in 0.05 point increments, with the umami intensity of water in test plot 1 as 0 point and the umami intensity of NC in test plot 2 as 0.30 points. The evaluation was performed by a trained panel of three, and the average score was used. The umami enhancement function of samples whose umami intensity was enhanced by 1.5 times or more (sodium glutamate 0.45 points or more) due to the addition was evaluated to be functional. In addition, the presence or absence, and intensity of off-taste (bitterness and harsh taste other than umami) were also evaluated. The evaluation results are shown in Table 12: Sensory evaluation (umami intensity).

**[Table 12]**

| | test plot | umami intensity (points) | degree of umami enhancement (times) | off-taste | off-taste intensity |
|---|---|---|---|---|---|
| 1 | water | 0 | - | - | - |
| 2 | NC | 0.30 | - | none | - |
| 3 | NC+Quercetin | 0.30 | 1.00 | bitter | + |
| 4 | NC+Luteolin | 0.43 | 1. 43 | none | - |
| 5 | NC+Pinocembrin | 0.50 | 1. 67 | none | - |
| 6 | NC+Liquiritigenin | 0.56 | 1.87 | none | - |
| 7 | NC+Apigenin | 0.56 | 1.87 | none | - |
| 8 | NC+Acacetin | 0.50 | 1.67 | none | - |

As in the above-mentioned results, the addition of four flavonoid components excluding quercetin and luteolin was found to show an effect of enhancing the umami intensity of an umami solution, without imparting an off-taste. However, luteolin was found to have a tendency to enhance umami. On the other hand, quercetin was found to have only the effect of imparting bitterness.

### [Industrial Applicability]

The present invention discloses, as a specific embodiment thereof, an agent for enhancing saltiness and/or umami of an oral ingestion product, which contains the present flavonoid component or a salt thereof as an active ingredient, and the like, and is useful, for example, in the food field.

This application is based on a patent application No. 2023-009728 filed in Japan (filing date: January 25, 2023), the contents of which are incorporated in full herein.

## Claims

1. An agent for enhancing saltiness and/or umami of an oral ingestion product comprising a component imparting saltiness and/or a component imparting umami, the agent comprising one or two or more flavonoid components selected from naringenin, luteolin, pinocembrin, liquiritigenin, apigenin, and acacetin, or salts thereof, as an active ingredient.

2. The agent according to claim 1, wherein the component imparting saltiness is one or two or more components selected from sodium chloride (NaCl), Na ion, and KCl.

3. The agent according to claim 1, wherein the component imparting umami is one or two or more components selected from glutamate, aspartate, inosinate, and guanylate.

4. The agent according to any one of claims 1 to 3, wherein the oral ingestion product is a food or drink, or a pharmaceutical product.

5. The agent according to any one of claims 1 to 3, which is in the form of a composition containing one or two or more flavonoid components selected from naringenin, luteolin, pinocembrin, liquiritigenin, apigenin, and acacetin, or a salt thereof.

6. The agent according to claim 5, which is in the form of a seasoning containing one or two or more flavonoid components selected from naringenin, luteolin, pinocembrine, liquiritigenin, apigenin, and acacetin, or a salt thereof.

7. The agent according to any one of claims 1 to 3, which is added to an oral ingestion product such that the content of one or two or more flavonoid components selected from naringenin, luteolin, pinocembrin, liquiritigenin, apigenin, and acacetin, or a salt thereof in the oral ingestion product is 0.01 weight ppm to 100 weight ppm as flavonoid component when orally ingested.

8. The agent according to any one of claims 1 to 3, wherein the flavonoid component is naringenin.

9. A method for producing an oral ingestion product having enhanced saltiness and/or umami, comprising adding the agent according to any one of claims 1 to 3 to an oral ingestion product containing a component imparting saltiness and/or a component imparting umami.

10. An oral ingestion product containing a component imparting saltiness and/or a component imparting umami (excluding oral ingestion products containing γ-aminobutyric acid), further comprising the agent according to any one of claims 1 to 3.

11. A method for enhancing the saltiness and/or umami of an oral ingestion product containing a component imparting saltiness and/or a component imparting umami, comprising adding one or two or more flavonoid components selected from naringenin, luteolin, pinocembrin, liquiritigenin, apigenin, and acacetin, or a salt thereof to the oral ingestion product.

12. The method according to claim 11, wherein the flavonoid component is naringenin.

13. One or two or more flavonoid components selected from naringenin, luteolin, pinocembrin, liquiritigenin, apigenin, and acacetin, or a salt thereof, which is used to enhance the saltiness and/or umami of an oral ingestion product containing a component imparting saltiness and/or a component imparting umami.

14. The flavonoid component or salt thereof according to claim 13, wherein the flavonoid component is naringenin.
